(19) Europäisches Patentamt / European Patent Office / Office européen des brevets

(11) **EP 1 535 568 B1**

(12) **EUROPEAN PATENT SPECIFICATION**

(45) Date of publication and mention
of the grant of the patent:
**19.11.2008 Bulletin 2008/47**

(51) Int Cl.:
*A61B 5/00* (2006.01)   *A61B 1/05* (2006.01)

(21) Application number: **05002895.0**

(22) Date of filing: **26.06.2002**

(54) **Apparatus for obtaining fluorescence images, and computer executable program therefor**

Gerät zur Herstellung von fluorezierenden Bildern und Computerausführbares Programm dafür

Appareil d'obtention d' images fluorescentes et programme correspondant exécutable par ordinateur

(84) Designated Contracting States:
**DE FR GB**

(30) Priority: **29.06.2001 JP 2001199131**
**27.03.2002 JP 2002089107**

(43) Date of publication of application:
**01.06.2005 Bulletin 2005/22**

(62) Document number(s) of the earlier application(s) in
accordance with Art. 76 EPC:
**02014010.9 / 1 275 338**

(73) Proprietor: **FUJIFILM Corporation**
**Minato-ku**
**Tokyo (JP)**

(72) Inventors:
• **Hayashi,Katsumi**
**Ashigarakami-gun, Kanagawa-Ken, Japan. (JP)**
• **The other inventor has agreed to waive his**
**entitlement to designation.**

(74) Representative: **Klunker . Schmitt-Nilson . Hirsch**
**Winzererstrasse 106**
**80797 München (DE)**

(56) References cited:
**WO-A-99/37204**      **US-A- 4 773 097**
**US-A- 5 131 398**     **US-A- 5 294 799**
**US-A- 5 769 792**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Field of the Invention

The present invention relates to a fluorescence image obtaining apparatus according to the preamble of claim 1. It also relates to a program for causing a computer to execute a fluorescence image obtaining method.

Description of the Related Art

Fluorescence detection apparatuses have been proposed that make use of the fact that the intensity of the fluorescence emitted from a normal tissue differs from the intensity of the fluorescence emitted from a diseased tissue when a target subject (i.e., a living tissue) is irradiated by an excitation light within an excitation wavelength range of the intrinsic fluorophores of the target subject. By detecting the fluorescence emitted from a target subject upon irradiation thereof by an excitation light within a predetermined wavelength range, the location and range of penetration of a diseased tissue is discerned.

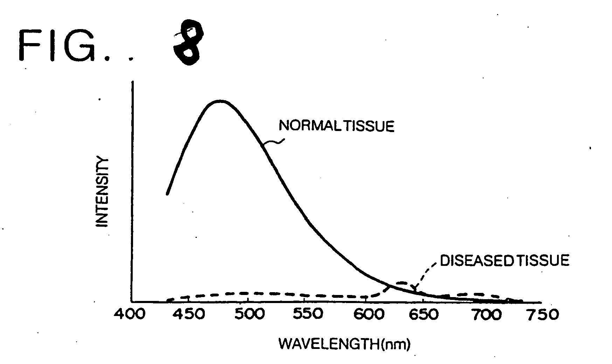

Normally, when a target subject is irradiated by an excitation light, because a high-intensity fluorescence is emitted from a normal tissue, as shown by the solid line in Fig. 8, and a weak-intensity fluorescence is emitted from a diseased tissue, as shown by the broken line in Fig. 8, by measuring the intensity of the fluorescence emitted from the target subject, it can be determined whether the target subject is in a normal or a diseased state.

Further, methods of imaging fluorescence by use of an imaging element or the like, and displaying a diagnostic fluorescence image corresponding to the intensity of the imaged fluorescence have been proposed. Here, because there is unevenness on the surface of a target subject, the intensity of the excitation light irradiating the target subject is not of a uniform intensity across the entirety of the surface thereof. Further, although the intensity of the fluorescence emitted from the target subject is substantially proportional to the intensity of the excitation light, the intensity of the aforementioned excitation light becomes weaker in inverse proportion to the square of the distance between the excitation light source and the target subject. Therefore, there are cases in which the fluorescence received from a diseased tissue located at a position closer to the excitation light source than a normal tissue is of a higher intensity than the fluorescence received from aforementioned normal tissue. Consequently, the state of the tissue of the target subject cannot be accurately discerned based solely on the data relating to the intensity of the fluorescence received from the target subject upon the irradiation thereof with an excitation light. In order to remedy the problems described above: a method of displaying an image based on the difference between the spectral forms representing the tissue states, that is, a method of dividing the intensities of two types of fluorescence intensities of two fluorescence images, each formed of fluorescence of a mutually different wavelength band (a narrow band near 480 nm and a wide band from near 430-730 nm) to obtain the ratio therebetween and displaying a computed image based on the obtained factor thereof as a fluorescence diagnostic image; a method of obtaining a value representing a fluorescence yield and displaying an image, that is, a method of projecting, as a reference light, onto a target subject a near-infrared light which is absorbed uniformly absorbed by tissues of a variety of tissue states, detecting the intensity of the reflected light reflected from the target subject upon the irradiation thereof by the reference light and dividing the intensity of the reflected light by the intensity of the fluorescence intensity to obtain the ratio therebetween, and displaying a computed image based on the obtained factor thereof as a fluorescence diagnostic image; and the like have been proposed. Further there have been proposed: a method of assigning color data to the factor of the intensities of the fluorescence of two different wavelength bands, or to the factor of a fluorescence intensity and the intensity of the reflected light reflected from the target subject upon the irradiation thereof by a reference light, to form a fluorescence diagnostic image wherein the diseased tissue of the target subject can be discerned from the difference in color within the fluorescence diagnostic image; a method of combining the color image representing the diseased tissue of the target subject by the difference in color and a brightness image formed by assigning brightness data to the intensity of the reflected light reflected from the target subject upon the irradiation thereof by the reference light to display a fluorescence diagnostic image also representing the contour of the surface of the target subject and imparting a three-dimensional sense thereof; such as those described in US Patent Nos. 5590660, 5647368, and Japanese Unexamined Patent Publication Nos. 9(1997)-308609, 10(1998)-225436, and 2001-157658.

In this manner, by obtaining, displaying on a monitor and observing a fluorescence diagnostic image, an accurate determination can be made as to whether the target subject is in a normal state or a diseased state.

However, for cases in which a fluorescence diagnostic image is obtained of a target subject when blood, mucus, digestive fluids, saliva, foam, residue, and/or the like (hereinafter referred to as obstructing factors) is present on the target subject, because the obstructing factor is also obtained in the image at the same time, the fluorescence diagnostic image contains an image of the obstructing factor. Here, if an obstructing factor is present on the target subject, the intensity of the fluorescence emitted from the portion on which the obstructing factor is present becomes reduced, and fluorescence of a wavelength greater than or equal to 600 nm is emitted. Therefore, if a diagnosis is carried out using a fluorescence diagnostic image including an obstructing factor, there is a fear that the portion on which the obstructing factor is present will be judged to be a diseased tissue, even though said portion is a normal tissue. Hereinafter, the reasons whereby an obstructing factor leads to misdiagnosis will be explained.

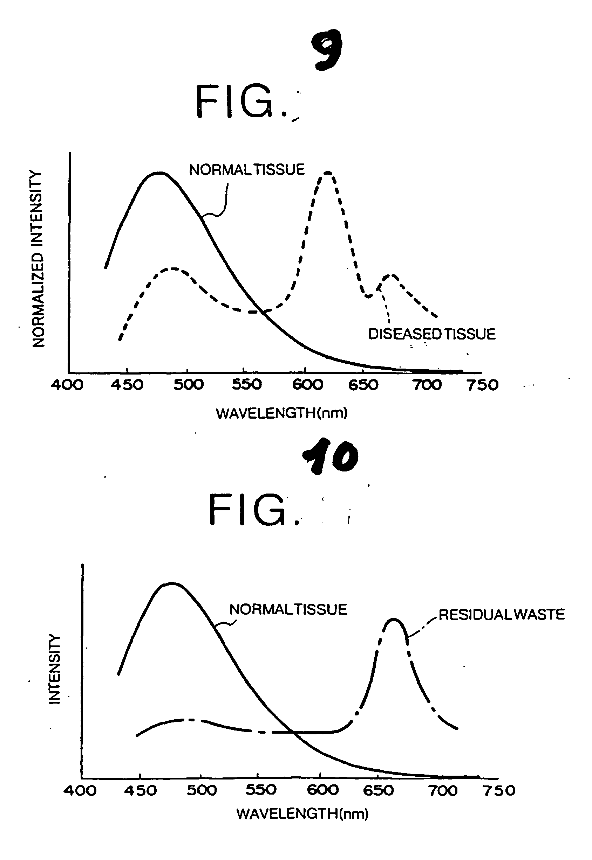

The spectrum of the fluorescence intensity emitted from the target subject upon the irradiation thereof by the excitation light is that shown in Fig. 8, and the normalized fluorescence intensity spectrum obtained by normalizing (causing the integral value over the entirety of the wavelength band to become 1) the aforementioned fluorescence intensity spectrum is that shown in Fig. 9. As shown in Fig. 8, the fluorescence intensity (an integral value over the entire wavelength band) emitted from a normal tissue and the fluorescence intensity emitted from a diseased tissue are clearly different. Further, as shown in Fig. 9, in the normalized fluorescence intensity spectrum, the relative intensity of the fluorescence of a wavelength near 480 nm emitted from the diseased tissue is reduced in comparison to that emitted from the normal tissue state, and the relative intensity of the fluorescence of a wavelength near 630 nm emitted from the diseased tissue is greater in comparison to that emitted from the normal tissue. Accordingly, it can be determined if the target subject is a normal tissue or a diseased tissue based on the fluorescence intensity and the normalized fluorescence intensity.

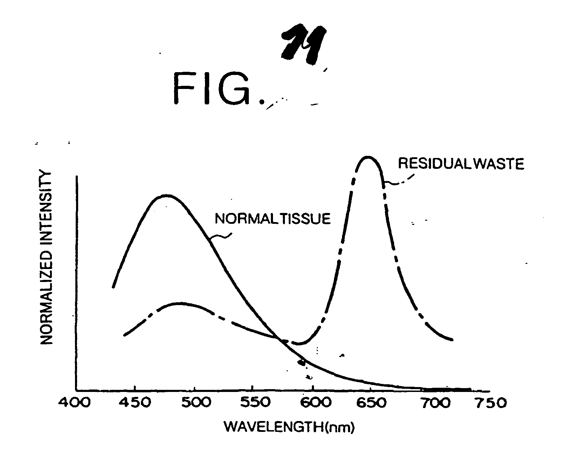

On the other hand, Fig. 10 shows the fluorescence intensity spectrum obtained of the fluorescence emitted from a residue obstructing factor upon the irradiation thereof by an excitation light, and Fig. 11 shows the normalized fluorescence intensity spectrum thereof. As shown in Fig. 10, in the case of residue, the fluorescence intensity spectrum thereof becomes approximately the same degree as that of the fluorescence emitted from a normal tissue; however, as shown in Fig. 11, with respect to the normalized fluorescence intensity spectrum of the fluorescence intensity spectrum of the fluorescence emitted from an obstructing factor, the relative intensity of the fluorescence of a wavelength near 480 nm is lower in comparison to that emitted from the normal tissue, and the relative intensity of the fluorescence of a wavelength near 670 nm is greater in comparison to that emitted from the normal tissue. Accordingly, according to the method wherein a computed image based on the factor of the intensities of two different types of fluorescence is employed as a fluorescence diagnostic image as described above, for example, in the case in which a fluorescence diagnostic image reflecting the form of the normalized fluorescence image intensity spectrum is obtained, because the pixel values of a portion in which a residue is present become the same pixel values as that of a diseased tissue, there is a fear that regardless of the fact that the tissue on which an obstructing factor is present is a normal tissue, said tissue will be diagnosed as being a diseased tissue.

In accordance with the preamble of claim 1, WO 99/37204 A discloses a fluorescence image obtaining apparatus, wherein fluorescence data are obtained for different wavelengths, i.e. blue fluorescence and red fluorescence.

SUMMARY OF THE INVENTION

The present invention has been developed in view of the forgoing circumstances, and it is an objective of the present invention to provide a fluorescence image obtaining apparatus, and a program capable of causing a computer to execute a fluorescence image obtaining method; wherein, an accurate diagnosis can be performed using a fluorescence diagnostic image.

This is achieved by the features of claim 1 and claim 5, respectively.

The referents of "fluorescence diagnostic image" can include: an image corresponding to the intensity of the fluorescence emitted from a target subject upon the irradiation thereof by an excitation light; an image representing the ratio between two types of fluorescence intensities obtained of two different wavelength bands; an image representing the ratio of the fluorescence intensity to the intensity of the reflected light reflected from the target subject upon the irradiation thereof by a reference light; an image formed by assigning color data to the ratio between the fluorescence intensities obtained of two different wavelength bands; an image formed by assigning color data to the ratio of the of fluorescence intensity to the reflectance intensity of the reflected light reflected from the target subject upon the irradiation thereof by a reference light; a synthesized image formed by combining a color image to which color data has been assigned and a brightness image obtained by assigning brightness data to the reflectance intensity of the reflected light reflected from the target subject upon the irradiation thereof by a reference light; or the like.

The referents of "color data" include, for example: the hue, saturation, and/or chromaticity (hue and saturation) of development color systems (HSB/HVC/Lab/Luv/La*b*/Lu*v* color spaces) or a mixed color system (an X,Y,Z color space); the color differences of a visible image signal representative of a TV signal (e.g., the IQ of the YIQ of an NTSC signal, the CbCr of an YCbCr, etc.); the combination ratio of a color signal (R, G, B or C, M, Y, G), etc.

The referents of "brightness data" include, for example: the luminosity or brightness of development color systems (HSB/HVC/Lab/Luv/La*b*/Lu*v* color spaces)or a mixed color system (an X, Y, Z color space) ; the brightness of a visible image signal representative of a TV signal (e.g., the Y of the YIQ of an NTSC signal, the CbCr of an YCbCr, etc.); etc.

The referents of "obstructing regions" are the regions representing locations of the target subject on which an obstructing factor such as blood, mucus, digestive fluids, saliva, foam, residue, and/or the like is present. The obstructing regions are regions of which there is a high probability of the misdiagnosis thereof as a diseased tissue, regardless of the fact that the tissue represented therein is in a normal tissue state. Note that according to the present invention, the regions of a fluorescence diagnostic image corresponding to obstructing regions as well as the regions of a standard image corresponding to obstructing regions are referred to as obstructing regions.

The fluorescence intensity and the computed fluorescence value representing the ratio between a plurality of fluorescence intensities obtained of different wavelength bands are used as the fluorescence data.

Further, in this case, based on either the fluorescence intensity or the computed fluorescence value (e.g., the fluorescence intensity), the suspected obstructing regions of the target subject can be detected, and

based on the other of either of the fluorescence intensity and the computed fluorescence value (e.g., the computed fluorescence value), the obstructing regions suspected can be detected.

Further, in this case, based on any one of the color data, the fluorescence intensity, or the computed fluorescence value (e.g., the color data), the suspected obstructing regions of the target subject can be detected, wherein

it is preferable that the obstructing regions of said suspected obstructing regions are detected based on one of the data other than that employed in the detection (e.g., the fluorescence intensity or the computed fluorescence value).

Further, according to the fluorescence image obtaining method of the present invention, the obstructing regions of the fluorescence diagnostic image can be subjected to an exceptional display process, and the fluorescence diagnostic image subjected to said exceptional display process can be displayed.

The expression "exceptional display process" refers to a process enabling the display of the fluorescence diagnostic image in a manner wherein each image of an obstructing region included therein can be recognized as such at a glance. More specifically, the images of the obstructing regions can be processed so as to be of a color not appearing in any of the images of the other regions. For example, if the fluorescence diagnostic image is an image having chromatic color, the images of the obstructing regions can be regions having achromatic color, or conversely, if the fluorescence diagnostic image is an image having achromatic color, the images of the obstructing regions can be regions having chromatic color; further, for a case in which the color of the fluorescence diagnostic image changes from a green through yellow color to red in correspondence to the change of the tissue state from normal to diseased, the images of the obstructing regions can be caused to be blue. Still further, the images of the obstructing regions can be caused to be the same color as the background, or transparent. In addition, the images of the regions included in the fluorescence diagnostic image other than the obstructing regions can be caused to be transparent. Also, although there are cases in which the portions regarded to be in the diseased state are indicated by an arrow mark, in this type of case, a process whereby arrow marks are not assigned to obstructing regions is included in the exceptional display processes.

Further, according to the fluorescence image obtaining apparatus of the present invention, it is preferable that an exceptional display process means for subjecting the obstructing regions of the fluorescence diagnostic image to exceptional display processes, and

a display means for displaying the fluorescence diagnostic image that has been subjected to said exceptional display processes be further provided.

Still further, according to the fluorescence image obtaining apparatus according to the present invention, it is preferable that a portion or the entirety of the fluorescence diagnostic image obtaining means be provided in the form of an endoscope to be inserted into the body cavity of a patient.

The fluorescence image obtaining method of the present invention may be provided as a program capable of causing a computer to execute said fluorescence image obtaining.method.

According to the present invention, when a fluorescence diagnostic image is obtained, because the obstructing regions representing the obstructing factor present on the target subject are detected, by causing the obstructing regions to be of a color different from that of the other regions or removing the obstructing regions, etc. and displaying the fluorescence diagnostic image, the fear that an obstructing region will be diagnosed as a tissue in a diseased state is eliminated. Accordingly, an accurate diagnosis can be performed using the fluorescence diagnostic image.

Further, for cases in which a standard image is obtained based on the reflected light obtained from the target subject upon the irradiation thereof by a white light, the obstructing regions included within the standard image become a different color than the other regions. Accordingly, the obstructing regions can be accurately detected based on the color data of the standard image.

Still further, for cases in which the fluorescence intensity emitted from the target subject including obstructing factors upon the irradiation thereof by an excitation light is obtained in a plurality of wavelength bands, and a computed fluorescence data representing the ratio between these plurality of fluorescence intensities has been obtained, the computed fluorescence value of the obstructing regions becomes close to that of a diseased tissue. On the other hand, the fluorescence intensity emitted from the obstructing factors present on the target subject becomes a value close to that of the fluorescence intensity emitted from a normal tissue. Accordingly, the obstructing regions can be distinguished from the other regions of the target subject, based on the fluorescence data such as the fluorescence intensity, the computed fluorescence value, or the like. Therefore, the obstructing regions can be accurately detected based on the fluorescence data.

In particular, when the obstructing regions are detected based on the fluorescence intensity and the computed fluorescence value, by detecting, based on either of the fluorescence intensity or the computed fluorescence value, the suspected obstructing regions, and further detecting, based on the other of either the fluorescence intensity or the computed fluorescence value, the obstructing regions of the suspected obstructing regions, the amount of computation required for detecting the obstructing regions can be reduced by the detection of the obstructing regions from the suspected obstructing regions compared to the case in which the obstructing regions are detected from the fluorescence data across the entire area of the target subject. For example, for a case in which the suspected obstructing regions have been detected based on the fluorescence intensity, if the obstructing regions are detected, based on the computed fluorescence value, for only the suspected obstructing regions, the amount of calculation required for performing the detection can be reduced. On the other hand, for a case in which the suspected obstructing regions have been detected based on the computed fluorescence value, if the obstructing regions are detected, based on the fluorescence intensity, for only the suspected obstructing regions, the amount of calculation required for performing the detection can be reduced. Accordingly, the amount of calculation required for detecting the obstructing regions can be reduced, and the obstructing regions can be detected at a higher speed.

Further, by subjecting the obstructing regions occurring in a fluorescence diagnostic image to an exceptional display process when the fluorescence diagnostic image is to be displayed, when the displayed fluorescence diagnostic image is displayed, the obstructing regions can be recognized as such at a glance. Accordingly, the fear that an obstructing region will be misrecognized as a tissue in a diseased state is eliminated, and the diagnosis can be performed more accurately using the fluorescence diagnostic image.

Still further, if a fluorescence diagnostic image in which the images of the regions other than the obstructing regions have been caused to be transparent is superposed over a standard image and displayed, when the displayed standard image is observed, the obstructing regions can be recognized as such at a glance. Accordingly, the fear that a tissue in a diseased state that appears within obstructing region will be overlooked is eliminated, and the accuracy with which the diagnosis can be performed using the fluorescence diagnostic image is improved a level.

BRIEF DESCRIPTION OF THE DRAWINGS

Figure 1 is a schematic drawing of the main part of a fluorescence endoscope apparatus implementing the fluorescence image obtaining apparatus according to an example not covered by the present invention,
Figure 2 is a schematic drawing of a CYG filter,
Figure 3 is a schematic drawing of a switching filter,
Figure 4 is a flowchart of the operation of the first example from the detection of the obstructing regions to the performance of the exceptional display process,
Figure 5 is a schematic drawing of the main part of a fluorescence endoscope apparatus implementing the fluorescence image obtaining apparatus according to the second example,
Figure 6 is a flowchart of the operation of the second example from the detection of the obstructing regions to the performance of the exceptional display process,
Figure 7 is a schematic drawing of the main part of a fluorescence endoscope apparatus implementing the fluorescence image obtaining apparatus according to a variation of the second example of the present invention,
Figure 8 is a graph illustrating the respective intensity distributions of the fluorescence intensity spectrum of a tissue in a normal state and a tissue in a diseased state,
Figure 9 is a graph illustrating the respective intensity distributions of the normalized fluorescence intensity spectrum of a tissue in a normal state and a tissue in a diseased state,
Figure 10 is a graph illustrating the respective intensity distributions of the fluorescence intensity spectrum of a tissue in a normal state and a residue, and
Figure 11 is a graph illustrating the respective intensity distributions of the normalized fluorescence intensity spectrum of a tissue in a normal state and a residue.

DESCRIPTION OF THE PREFERRED EMBODIMENTS

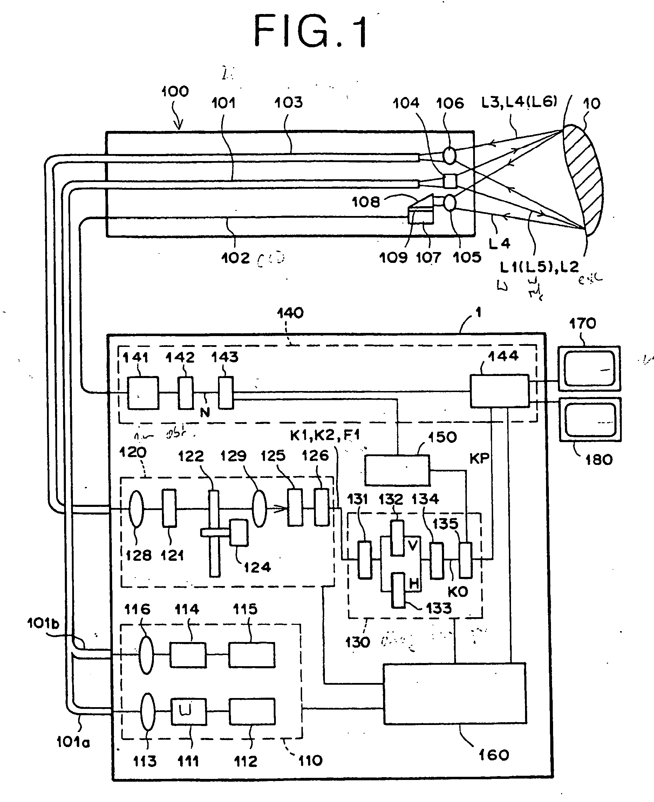

Before describing preferred embodiments of the present invention with reference to the attached drawings, examples will be described which are not part of the claimed invention. Fig. 1 is a schematic drawing of the main part of a fluorescence endoscope apparatus implementing the fluorescence image obtaining apparatus according to a first example. According to the fluorescence endoscope apparatus of the first example: the fluorescence emitted from a target subject upon the irradiation thereof by an excitation light is two-dimensionally detected by an image fiber; a narrow band fluorescence image formed of the fluorescence of a wavelength in the 430-530 nm wavelength band and a wide band fluorescence image formed of the fluorescence of a wavelength in the 530-730 nm wavelength band are obtained; a color image is formed based on the intensities of both fluorescence images, that is, on the factor of each corresponding pixel value of the narrow band fluorescence image and the wide band fluorescence image; an IR reflectance image is obtained of the reflected light reflected from the target subject upon the irradiation thereof by white light; a luminosity image is formed based on the light intensity of the IR reflectance image, that is, on the pixel value of each pixel of the IR reflectance image; the color image and the IR luminosity image are combined to form a synthesized image; and the synthesized image is displayed on a monitor as a fluorescence diagnostic image.

As shown in Fig. 1, the fluorescence endoscope apparatus according to the first example comprises: an endoscope insertion portion 100 for insertion into the primary nidus and suspected areas of disease of the patient; and an image signal processing portion 1.

The image signal processing portion 1 comprises: an illuminating unit 110 equipped with a light source for emitting a white light L1. (including a reference light L5) for obtaining a standard image and an IR reflectance image, and an excitation light L2 for obtaining a fluorescence image; an image obtaining unit 120 for obtaining two types of fluorescence images formed of different wavelength bands of fluorescence and an IR reflectance image of a target subject 10, and obtaining fluorescence image data K1, K2, and an IR reflectance image data F1; a fluorescence diagnostic image forming unit 130 for obtaining a factor between the corresponding pixel values of the respective fluorescence images represented by each of fluorescence image data K1 and K2 and obtaining a color image data H based on the obtained factor, forming a luminosity image data V based on the pixel value of the IR reflectance image represented by the IR reflectance image data F1, combining the color image data H and the luminosity image data V to form a fluorescence diagnostic image data, and further subjecting the fluorescence diagnostic image data to an exceptional display process, which is described below, to obtain a processed fluorescence diagnostic image data KP representing a processed fluorescence diagnostic image; an image processing unit 140 for subjecting the standard image represented by the standard image data N and the processed fluorescence diagnostic image represented by the processed fluorescence diagnostic image data KP to the processes required to display said images as visible images; an obstructing region detecting unit 150 for detecting the obstructing regions, which are described below; a controller 160 connected to each of the above units for controlling the operation timings thereof; a monitor 170 for displaying the normal image data N processed by the image process portion 140 as a visible image; and a monitor 180 for displaying the processed fluorescence diagnostic image data KP processed by the image process portion 140 as a visible image.

The endoscope insertion portion 100 is provided with a light guide 101 extending internally to the distal end thereof, A CCD cable 102, and an image fiber 103. An illuminating lens 104 and an objective lens 105 are provided at the distal end of the light guide 101, that is, at the distal end of the endoscope insertion portion 100. Further, the image fiber 103 is a quartz glass fiber, and is provided at the distal end thereof with a condensing lens 106. A CCD imaging element 107 (not shown) which is provided with an on-chip color filter is connected to the distal end of the CCD cable 102, and a prism 108 is attached to the CCD imaging element 107. Still further, an RGB filter 109 provided with R, G, and B band filter elements corresponding to each pixel of the CCD imaging element 107 and which are distributed in a mosaic pattern is disposed between the CCD imaging element 107 and the prism 108. A white light guide 101a, which is a composite glass fiber, and an excitation light guide 101b, which is a quartz glass fiber are bundled to form the light guide 101 as an integrated cable. The white light guide 101a and the excitation light guide 101b are connected to the illuminating unit 110. One end of the CCD cable 102 is connected to the image processing unit 140, and one end of the image fiber 103 is connected to the image obtaining unit 120.

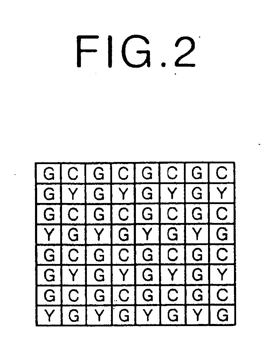

Note that a CYG filter, such as that shown in Fig. 2, which is formed of a C (cyan), a Y (yellow), and a G (green) band pass filters can be used instead of the RGB filter formed of the R, G, B band pass filters.

The illuminating unit 110 comprises: a white light source 111, which is a halogen lamp or the like, for emitting white light L1 (including a reference light L5 formed of near-infrared light) for obtaining standard images and IR reflectance images; a white light power source 112 which is electrically connected to the white light source 111; a white light condensing lens 113 for focusing the white light L1 emitted from the white light source 111; a GaN semiconductor laser 114 for emitting excitation light L2 for obtaining fluorescence images; an excitation light power source 115 which is electrically connected to the GaN semiconductor laser 114; and an excitation light condensing lens 116 for focusing the excitation light L2 emitted from the GaN semiconductor laser 114. Note that a reference light source that emits the reference light L5 can be provided separate from the white light source.

The image obtaining unit 120 comprises: a collimator lens 128 that guides the fluorescence L3 conveyed thereto via the image fiber 103; an excitation light cutoff filter 121 that cuts off light having a wavelength less than or equal to the 420 nm wavelength of the excitation light L2 from the fluorescence L3; a switching filter 122, in which three types of optical transmitting filters are combined; a filter rotating apparatus 124, which is a motor or the like, for rotating the switching filter 122; a condensing lens 129 for focusing the fluorescence L3 and the reflected light L6 transmitted by the switching filter 122; a CCD imaging element 125 for obtaining the fluorescence image and the IR reflectance image represented by the fluorescence L3 and the reflected light L6, respectively, focused by the condensing lens 129; and an A/D conversion circuit 126 for digitizing the image signals obtained by the CCD imaging element 125 to obtain two types of fluorescence image data K1, K2, and an IR reflectance image data F1.

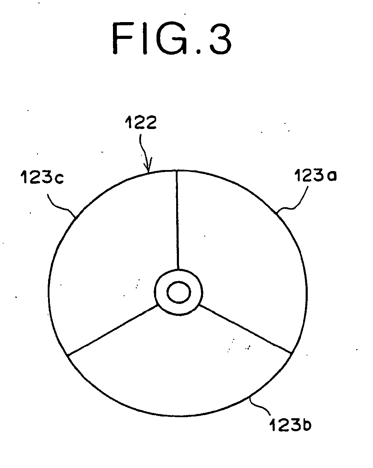

The configuration of the switching filter 122 is shown in Fig. 3. As shown in Fig. 3, the switching filter 122 comprises : an optical filter 123a, which is a band pass filter, that transmits light of a wavelength in the 430-730 wavelength band; an optical filter 123b, which is a band pass filter, that transmits light of a wavelength of 480 nm $\pm$50nm; an optical filter 123c, which is a band pass filter, that transmits light of a wavelength in the 750-900 wavelength band. The optical filter 123a is an optical filter for obtaining a wide band fluorescence image; the optical filter 123b is an optical filter for obtaining a narrow band fluorescence image, and the optical filter 123c is an optical filter for obtaining an IR reflectance image. The switching filter 122 is controlled by the controller 160 via the filter rotating apparatus 124 so that the optical filter 123c is disposed along the optical path when the target subject 10 is being irradiated by the white light L1; and the optical filters 123a and 123b are alternately disposed along the optical path when the target subject 10 is being irradiated by the excitation light L2.

The fluorescence diagnostic image forming means 130 comprises: an image memory 131 for storing the two types of fluorescence image data K1, K2, and the IR reflectance image data F1 obtained by the A/D conversion circuit 126; a luminosity image computing portion 132, in which a look up table correlating the range of each pixel value of the IR reflectance image represented by the IR reflectance image data F1 to a luminosity in a Munsel display color system is stored, for referring to said look up table and obtaining a luminosity image data V from the IR reflectance image data F1; a hue computing portion 133, in which a look up table correlating the range of the factor between the two types of fluorescence images represented by the fluorescence image data K1, K2, to a hue in the hue circle of a Munsel display color system is stored, for referring to said look up table and forming a hue image data H from the factor between said fluorescence images; an image synthesizing portion 134 for combining the hue image data H and the luminosity image data V to form a fluorescence diagnostic image data KO representing a fluorescence diagnostic image; and an exceptional display processing portion 135 for subjecting the obstructing portions of the fluorescence diagnostic image to an exceptional display process to obtain a processed fluorescence diagnostic data KP.

The image memory 131 comprises a narrow band fluorescence image data storage region, a wide band fluorescence image data storage region, and an IR reflectance image data storage region, which are not shown in the drawing, wherein: the narrow band fluorescence image data K1 representing the narrow band fluorescence image obtained in the state wherein the excitation light L2 is being emitted and the narrow band fluorescence image optical filter 123a is disposed along the optical path of the fluorescence L3 conveyed by the image fiber 103 is recorded in the narrow band fluorescence image storage region; and the wide band fluorescence image data K2 representing the wide band fluorescence image obtained in the state wherein the excitation light L2 is being emitted and the wide band fluorescence image optical filter 123b is disposed along the optical path of the fluorescence L3 conveyed by the image fiber 103 is recorded in the wide band fluorescence image storage region. Further, the IR reflectance image data K1 representing the IR reflectance image obtained in the state wherein the reference light L5, that is the white light L1, is being emitted and the IR reflectance image optical filter 123c is disposed along the optical path of the reflected light L6, that is, the reflected light L4 conveyed by the image fiber 103, is recorded in the IR reflectance image storage region.

The exceptional display processing portion 135 performs an exceptional display process on the obstructing regions of the fluorescence diagnostic image represented by the fluorescence diagnostic image data K0. The exceptional display process is a process that causes the obstructing regions of the fluorescence diagnostic image to be displayed in a different form with respect to the other regions of the fluorescence diagnostic image. More specifically, the pixel values corresponding to the obstructing regions are converted to a color not appearing in any of the other regions of the fluorescence diagnostic image. For example, the pixels values of the obstructing regions can be converted to a blue color for a case in which the color change of the normal tissue and the diseased tissue of the target subject 10 range from green through yellow to red. Note that the color of the obstructing regions can be caused to be the same color as the background color, or the obstructing regions can be caused to be transparent. Alternatively, the images of the regions other than the obstructing regions included in the fluorescence diagnostic image can be caused to be transparent. Further, according to the current embodiment, because the fluorescence diagnostic image is a chromatic color image, the obstructing regions can also be caused to be non-chromatic in color. Note that for cases in which the fluorescence diagnostic image is a non-chromatic image, the obstructing regions can be cased to be chromatic.

Still further, the pixels within the obstructing regions can be displayed as gradation values. More specifically, the average color value Cave obtained of the target subject 10 and the standard deviation Cstd can be computed in advance, and the Mahalanobis distance Cm for the pixel value Cxy of each pixel of the obstructing regions can be obtained according to the following formula (1):

$$Cm = (Cxy - Cave)^2 / Cstd$$

The Mahalanobis distance Cm obtained by the formula (1) increases as the possibility of an obstructing region being of a color other than the average color of the target subject 10 becomes higher. Accordingly, by assigning a gradation value to the value of the Mahalanobis distance Cm, the obstructing region can be displayed as a gradation image corresponding to the magnitude of the possibility that said obstructing region represents an obstructing factor. Note that instead of the gradation display, it is possible to set and display the obstructing regions at the contour lines corresponding, to the Mahalanobis distance Cm.

Further, for cases in which the portions regarded to be diseased tissue are indicated by an arrow mark, in the case of this type of display, a process whereby arrow marks are not assigned to obstructing regions is included in the exceptional display processes.

Note that the fluorescence diagnostic image forming unit 130 can be a unit for forming a processed fluorescence diagnostic image data KP based on the factor obtained between the corresponding pixel values of the fluorescence diagnostic images represented by the fluorescence diagnostic image data K1, K2, or based on the factor obtained by the performance of a division calculation between the pixel values of either of the fluorescence images and the pixel values of the IR reflectance image. Further, color data can be assigned to the factor obtained between the two fluorescence images or between one of the fluorescence images and the IR reflectance image, and the fluorescence diagnostic image data KP can be formed so as to represent the diseased state of the target subject 10 by the differences in color.

Further, for cases in which the IR reflectance image data F1 is used to form the fluorescence diagnostic image data K0, the R color data included in the standard image data N or the brightness data computed from the standard image data N can be used instead of the IR reflectance image data F1. Still further, for cases in which light of each of the colors R, G, and B is projected onto the target subject 10 and a standard image is obtained of the reflected light reflected from the target subject 10 thereupon, as described below, the color data based on the reflected red light can be used instead of the IR reflectance image data F1.

The image processing unit 140 comprises a signal processing circuit 141 for forming an analog standard image signal of the standard image, which is a color image, represented by the signal obtained by the CCD imaging element 107; an A/D converting circuit 142 for digitizing the standard image data formed in the signal processing circuit 141 to obtain a digital standard image data N; a standard image memory 143 for storing the standard image data N; and a video signal processing circuit 144 for converting the standard image data N outputted from the standard image memory 143 and the processed fluorescence diagnostic image data KP formed in the fluorescence diagnostic image forming unit 130 to video signals.

The obstructing regions detecting unit 150 is means that detects, based on the color data of the standard image represented by a standard image data N, obstructing regions representing regions in which an obstructing factor, such as blood, mucus, digestive fluids, saliva, foam, residue and/or the like is present on the target subject 10. Here, the color data can be that of, for example: the hue, saturation, and/or chromaticity (hue and saturation) of development color systems (HSB/HVC/Lab/Luv/La*b*/Lu*v* color spaces) or a mixed color system (an X,Y,Z color space); the color differences of a visible image signal representative of a TV signal (e.g., the IQ of the YIQ of an NTSC signal, the CbCr of an YCbCr, etc.); the combination ratio of a color signal (R, G, B or C, M, Y, G), etc.

More specifically, for the case in which the hue data is used as the color data, the standard image is of a specific hue range for cases in which the target subject 10 is a normal tissue and for cases in which the target subject 10 is a diseased tissue, respectively. On the other hand, for cases in which obstructing regions are present in the standard image, the hue of the obstructing factors is a hue other than that of either a normal tissue or a diseased tissue. Accordingly, the hue of each pixel of a standard image based on a standard image data N is computed, and a determination is made as to whether or not the hue of each pixel is the outside of a predetermined specific range; regions formed of pixels having a hue outside the predetermined specific range are detected as obstructing regions.

Further, for the case in which the chromaticity is used as the color data, the standard image is of a specific chromaticity range on the chromaticity chart for cases in which the target subject 10 is a normal tissue and for cases in which the target subject 10 is a diseased tissue, respectively. On the other hand, for cases in which obstructing regions are present in the standard image, the chromaticity of the obstructing factors is a chromaticity other than that of a normal tissue or a diseased tissue. Accordingly, the chromaticity of each pixel of a standard image based on a standard image data N is computed; and a determination is made as to whether or not the chromaticity of each pixel is the outside of a predetermined specific range; regions formed of pixels having a chromaticity outside the predetermined specific range are detected as obstructing regions.

Note that because the standard image data N is data formed of the data of each color R, G, B (or C, Y, G); the hue and chromaticity can be easily obtained if each color data is used. On the other hand, for the case in which the obstructing regions are detected based on the difference in color, the color difference signal can be computed from each color data R, G, B (or C, Y, G). However, according to the video signal processing circuit 144 according to the current embodiment, the standard image data N is converted to a video signal formed of brightness signals and color difference signals. Accordingly, if the color difference is to be used as the color data, the color difference obtained by the conversion of the standard image data N to a video signal by the video signal processing circuit 144 is used thereas; by the detection

EP 1 535 568 B1

of the obstructing pixels by the obstructing region detecting unit 150, the step wherein the color difference is computed by the obstructing regions detecting unit 150 can be omitted.

Next, the operation of the first example will be explained. First, the operation occurring when a standard image is to be obtained and displayed will be explained, followed by an explanation of the operations occurring when a reflectance image and a fluorescence image are to be obtained, and then an explanation of the operations occurring when the obstructing regions are detected, the fluorescence diagnostic image is synthesized, and the processed fluorescence diagnostic image is displayed will be explained.

According to the first example, the obtainment of a standard image, an IR reflectance image, and a fluorescence image are performed alternately in a temporal series. When the standard image and the IR reflectance image are to be obtained, the white light source power source 112 is activated, based on a signal from the controller 160, and white light L1 is emitted from the white light source 111. The white light L1 is transmitted by the white light condensing lens 113 and enters the white light guide 101a, and after being guided to the distal end of the endoscope insertion portion 100, is projected onto the target subject 10 from the illuminating lens 104.

The reflected light L4 of the white light L1 is focused by the objective lens 105, reflected by the prism 108, transmitted by the RGB filter 109, and focused on the CCD imaging element 107.

The signal processing circuit 141 forms an analog standard image signal, which represents a color image, from the reflected light L4 imaged by the CCD imaging element 107. The analog standard image signal is inputted to the A/D converting circuit 142, and after being digitized therein, is stored in the standard image memory 143. The standard image data N stored in the standard image memory 143 is converted to a video signal by the video signal converting circuit 144, and then input to the monitor 170 and displayed thereon as a visible image. The series of operations described above are controlled by the controller 160.

Meanwhile, at the same time, the reflected light L4 of the white light L1 (including the reflected light L6 of the reference light L5) is focused by the condensing lens 106, enters the distal end of the image fiber 103, passes through the image fiber 103 and is focused by the collimator lens 128, and is transmitted by the excitation light cutoff filter 121 and the optical filter 123c of the switching filter 122.

Because the optical filter 123c is a band pass filter that only transmits light of a wavelength in the 750-900 nm wavelength band, only the reflected light L6 of the reference light L5 is transmitted by the optical filter 123c.

The reflected light L6 transmitted by the optical filter 123c is received by the CCD imaging element 125. The analog IR reflectance image data obtained by the photoelectric conversion performed by the CCD imaging element 125 is digitized by the A/D converting circuit 126, and then stored as an IR reflectance image data F1 in the IR reflectance image region of the image memory 131 of the fluorescence image forming unit 130.

Next, the operation occurring when the fluorescence image is to be obtained will be explained. The excitation light source power source 115 is activated, based on a signal from the controller 160, and a 410 nm wavelength excitation light L2 is emitted from the GaN type semiconductor laser 114. The excitation light L2 is transmitted by the excitation light condensing lens 116 and enters the excitation light guide 101b, and after being guided to the distal end of the endoscope insertion portion 100, is projected onto the target subject 10 from the illuminating lens 104.

The fluorescence L3 emitted from the target subject 10 upon the irradiation thereof by the excitation light L2 is focused by the condensing lens 106, enters the distal end of the image fiber 103, passes through the image fiber 103 and is focused by the collimator lens 128, and is transmitted by the excitation light cutoff filter 121 and the optical filters 123a and 123b of the switching filter 122.

Because the optical filter 123a is a band pass filter that only transmits light of a wavelength in the 430-730 nm wavelength band, the fluorescence L3 transmitted by the optical filter 123a represents a wide band fluorescence image. Because the optical filter 123b is a band pass filter that only transmits light of a wavelength of $480 \pm 50$ nm, the fluorescence L3 transmitted by the optical filter 123b represents a narrow band fluorescence image.

The fluorescence L3 representing the narrow band fluorescence image and the wide band fluorescence image is received by the CCD imaging element 125, photoelectrically converted thereby, digitized by the A/D converting circuit 126, and then stored as a wide band fluorescence image data K1 in the wide band fluorescence image region and a narrow band fluorescence image data K2 the narrow band fluorescence image region of the image memory 131 of the fluorescence image forming unit 130.

Hereinafter the operation occurring when a processed fluorescence diagnostic image data KP is to be formed by the fluorescence diagnostic image forming unit 130 will be explained. First, the luminosity image computing portion 132 determines, utilizing the signal charge and a look up table, a luminosity occurring in a Munsel display color system for each pixel value of the IR reflectance image represented by the IR reflectance image data F1 to obtain a luminosity image data V, and outputs said luminosity image data V to the image synthesizing means 134.

The hue computing portion 133 of the fluorescence diagnostic image forming unit 130 divides the pixel value of each pixel of the narrow band fluorescence image represented by the narrow band fluorescence image data K2 by the pixel value of each corresponding pixel of the wide band fluorescence image represented by the in the wide band fluorescence image data K1 stored in the image memory 131 to obtain the respective factors thereof, and obtains, utilizing said factors and a prerecorded lookup table, a hue occurring in a Munsel display color system to obtain a hue image data H, and outputs the hue image data H to the image synthesizing portion 134.

The image synthesizing portion 134 synthesizes the hue image data H and the luminosity image data V to form a fluorescence diagnostic image K0 representing a fluorescence diagnostic image. Note that for cases in which the image is to be displayed in color, the image is displayed as a three color image; because the hue, luminosity, and saturation are required, when the image is synthesized, the largest values of the hue and the luminosity are obtained as the saturation S occurring in a Munsel display color system. Note that the fluorescence diagnostic image data K0 is subjected to an RGB conversion process, and becomes an image representing each of color R, G, and B.

Meanwhile, the obstructing regions detecting unit 150 detects, based on the color data of the standard image represented by the standard image data N, the regions of the target subject 10 on which an obstructing factor is present. Then, the exceptional display process unit 135 of the fluorescence diagnostic image forming unit 130 subjects the obstructing regions of the fluorescence diagnostic image represented by the fluorescence diagnostic image data K0 to an exceptional display process to obtain a processed fluorescence diagnostic image data KP.

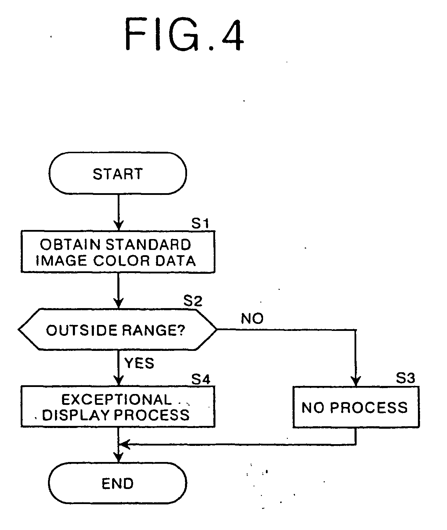

Hereinafter, the operations occurring from the detection of the obstructing regions to the performance of the exceptional display process will be explained utilizing the flowchart of Fig. 4. Fig. 4 is a flowchart of the operations occurring from the detection of the obstructing regions to the performance of the exceptional display process. First, the color data of each pixel of the standard image is computed by the obstructing regions detecting unit 150 (step S1), and then, a determination is made as to whether or not the color data obtained of each pixel of the standard image is outside a predetermined range (step S2). If the result of the determination made in step S2 is a negative, because the pixel of which a negative result is obtained is not a pixel representing an obstructing region, the corresponding pixel thereto of the fluorescence diagnostic image is subjected to no process whatsoever (step S3). If the result of the determination made in step S2 is a positive, the pixel of which a positive result is obtained is recognized as a pixel representing an obstructing region, and the corresponding pixel thereto of the fluorescence diagnostic image represented by the fluorescence diagnostic image data K0 is subjected to the exceptional display process by the exceptional display: process portion 135 of the fluorescence diagnostic image forming unit 130 to obtain a processed fluorescence diagnostic image data KP (step S4).

The processed fluorescence diagnostic image data KP is outputted to the video signal processing circuit 144 of the image processing unit 140. The processed fluorescence diagnostic image data KP which has been converted to a video signal by the video signal processing circuit 144 is inputted to the monitor 180 and displayed thereon as a visible image. The obstructing regions of the processed fluorescence diagnostic image displayed on the monitor 180 have been subjected to the exceptional display process.

In this manner, the according to the current example, because the obstructing regions within the fluorescence diagnostic image have been detected, by displaying on the monitor 180 the processed fluorescence diagnostic image obtained by subjecting the detected obstructing regions therein to an exceptional display process, the obstructing regions included in the fluorescence diagnostic image can be recognized in at a glance. Accordingly, an accurate diagnosis can be performed utilizing the fluorescence diagnostic image with no fear that obstructing regions will be diagnosed to be diseased tissue.

Further, if the exceptional display process consists of subjecting the images other than the obstructing regions occurring in the fluorescence diagnostic image to a process whereby said other regions are rendered transparent to obtain a processed fluorescence diagnostic image, and said obtained processed fluorescence diagnostic image is superposed over the standard image and displayed on the monitor 180, by observing said displayed standard image, the obstructing regions included therein can be recognized as such at a glance. Accordingly, the fear that a tissue in a diseased state appearing within an obstructing region will be overlooked is eliminated, and the accuracy with which the diagnosis can be performed using the fluorescence diagnostic image is improved a level.

Still further, if a configuration is adopted wherein the exceptional display process is capable of being selected, by use of an external switch or the like, from a plurality of exceptional display processes, the operational ease and versatility can be improved a level. In when a standard diagnosis, for example, is to be performed, by displaying a fluorescence diagnostic image in which the obstructing regions included therein are of achromatic color, and the other portions thereof are of chromatic color, the misdiagnosis of obstructing regions as diseased tissue is prevented; on the other hand, by subjecting the images other than the obstructing regions occurring in the fluorescence diagnostic image to a process whereby said other regions are rendered transparent to obtain a processed fluorescence diagnostic image, and superposing said obtained processed fluorescence diagnostic image over the standard image immediately prior to concluding the diagnosis, the overlooking of diseased tissue included within the obstructing regions can be prevented.

Further, because the color of the obstructing regions differs from the color of the other regions, by detecting the obstructing regions based on the color data of the standard image, the obstructing regions can be detected accurately.

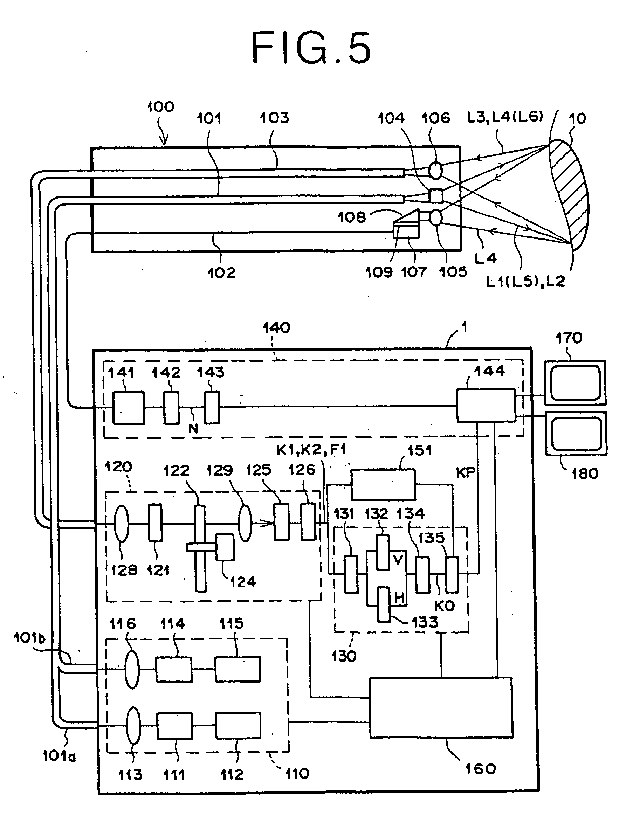

Next, an embodiment of the present invention will be explained. Fig. 5 is a schematic drawing of the main part of a fluorescence endoscope apparatus implementing the fluorescence image obtaining apparatus according to the embodiment of the present invention. Note that elements of the embodiment that are the same as those of the first example are likewise labeled, and further explanation thereof omitted. As shown in Fig. 5, the fluorescence endoscope apparatus according to the embodiment of the present invention differs from that of the above first example in that instead of the obstructing regions detecting unit 150, which detects the obstructing regions based on the color data of the standard image, an obstructing regions detecting unit 151, which detects the obstructing regions based on the fluorescence intensity and the factor, that is the ratio between the pixel values of the corresponding pixels of two fluorescence images represented by two fluorescence image data K1, K2, respectively, is provided.

Here, the factor (hereinafter referred to as the computed fluorescence value) of the corresponding pixel values between the fluorescence images represented by the fluorescence image data K1 and K2 for an obstructing region is smaller than the value obtained of normal tissue and is close to the value obtained of a diseased tissue. On the other hand, the fluorescence intensity of an obstructing region is close to that of a normal tissue. Accordingly, the obstructing regions detecting unit 151 obtains the computed fluorescence value from the fluorescence image data K1 and K2, and makes a determination as to whether or not the obtained computed fluorescence value is less than or equal to a predetermined threshold value Th1. Next, a determination is made with respect to only the pixels of which the pixel value thereof has been determined to be less than or equal to the threshold value Th1, as to whether or not the fluorescence intensity thereof, that is, the fluorescence intensity of the pixel values of the fluorescence image represented by the fluorescence image data K1 or K2 is greater than or equal to a second threshold value Th2; the pixels of which the fluorescence intensity is determined to be greater than or equal to the threshold value Th2 are detected as obstructing regions. Note that instead of obtaining the computed fluorescence value itself, the obstructing regions detecting unit 151 can utilize the factor obtained of the corresponding pixels between the fluorescence images by the hue computing means 133 of the fluorescence diagnostic image forming means 130.

Next, the operation of the embodiment will be explained. The operations occurring when the standard image is to be obtained, the standard image is to be displayed, the IR reflectance image is to be obtained, the fluorescence images are to be obtained, and the fluorescence diagnostic image is to be synthesized are the same as those occurring in the first example; therefore, further explanation thereof is omitted. The operations occurring when the obstructing regions are to be detected and the processed fluorescence diagnostic image is to be displayed will be explained.

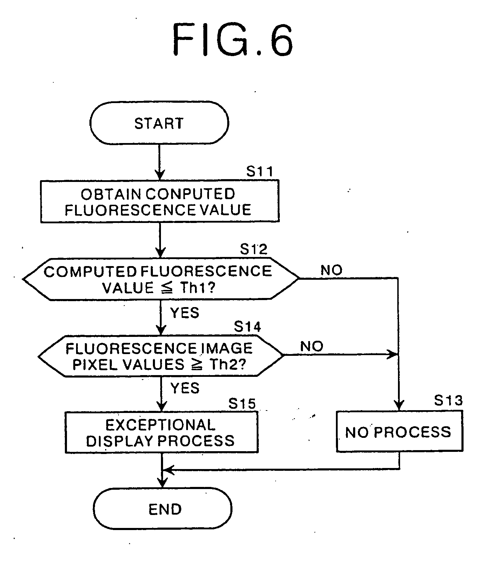

Fig. 6 is a flowchart of the operations from the detection of the obstructing regions to the performance of the exceptional display process according to the embodiment. As shown in Fig. 6: first, the ratio between the fluorescence images represented by the fluorescence image data K1 and K2, that is, the computed fluorescence value therebetween, is obtained by the obstructing regions detecting unit 151 (step S11); then, a determination as to whether or not the computed fluorescence value of each pixel of the fluorescence images is less than or equal to the threshold value Th1 (step S12). If the result of the determination made in step S12 is a negative, because the pixel of which a negative result is obtained is not a pixel representing an obstructing region, the corresponding pixel thereto of the fluorescence diagnostic image is subjected to no process whatsoever (step S13). If the result of the determination made in step S12 is a positive, because the possibility is high that the pixel of which a positive result is obtained is a pixel representing an obstructing region, a determination is made as to whether or not the fluorescence intensity thereof is greater than or equal to the threshold value Th2 (step S14). If the result of the determination made in step S14 is a negative, because the pixel of which a negative result is obtained is not a pixel representing an obstructing region, the corresponding pixel thereto of the fluorescence diagnostic image is subjected to no process whatsoever (step S13). If the result of the determination made in step S14 is a positive, the pixel of the fluorescence image represented by the respective fluorescence image data K1 or K2 is detected as an obstructing region, and the corresponding fluorescence diagnostic image data K0 is subjected to the exceptional display process by the exceptional display process portion 135 of the fluorescence diagnostic image forming unit 130 to obtain a processed fluorescence diagnostic image data KP (step S15).

The processed fluorescence diagnostic image data KP is outputted to the video signal processing circuit 144 of the image processing unit 140, and displayed on the monitor 180 as a visible image in the state in which the obstructing regions of the processed fluorescence diagnostic image have been subjected to the exceptional display process, in the same manner as occurred in the first embodiment.

Note that according to the embodiment, although the determination performed in step 14 as to whether or not the pixel value is greater than or equal to the threshold value Th2 is performed only on the pixels of which the computed fluorescence value has been determined to be less than or equal to the threshold value Th1 is the step S12, the determination of step S14 can be performed first, and the computed fluorescence value obtained and the process of step S11 and the determination of S12 performed only for pixels that have returned a positive result in step S14. Further, the process of step S11, the determination of step S12, and the determination of step S14 can be performed in a series for all pixels, and the pixels of which the computed fluorescence value is less than or equal to the threshold value Th1 and which also have a pixel value greater than or equal to the threshold value Th2 detected as obstructing regions.

Further, according to the embodiment, for cases in which the pixels within the obstructing regions are to be displayed with a display gradation: first, the average value FL ave of the fluorescence intensity obtained of the target subject 10 and the standard deviation FL std are computed in advance, and the Mahalanobis distance Fm of each pixel value FL xy included in the obstructing regions is obtained according to the formula (2) below.

$$Fm = (FLxy - Flave)^2 / FL\ std\ (2)$$

The length of the Mahalanobis distance Fm obtained by use of the formula (2) becomes longer in proportion to an increase in the possibility that the fluorescence intensity is that of an obstructing region, which deviates from the average fluorescence intensity of the target subject 10. Accordingly, by assigning a gradation to the value of the Mahalanobis distance Fm, the obstructing regions can be displayed with a display gradation corresponding to the increase in the possibility that the obstructing region represents an obstructing factor. Note that instead of employing the display gradation, a contour line can be set in the obstructing regions in correspondence to the length of the Mahalanobis distance Fm, and displayed as a contour display.

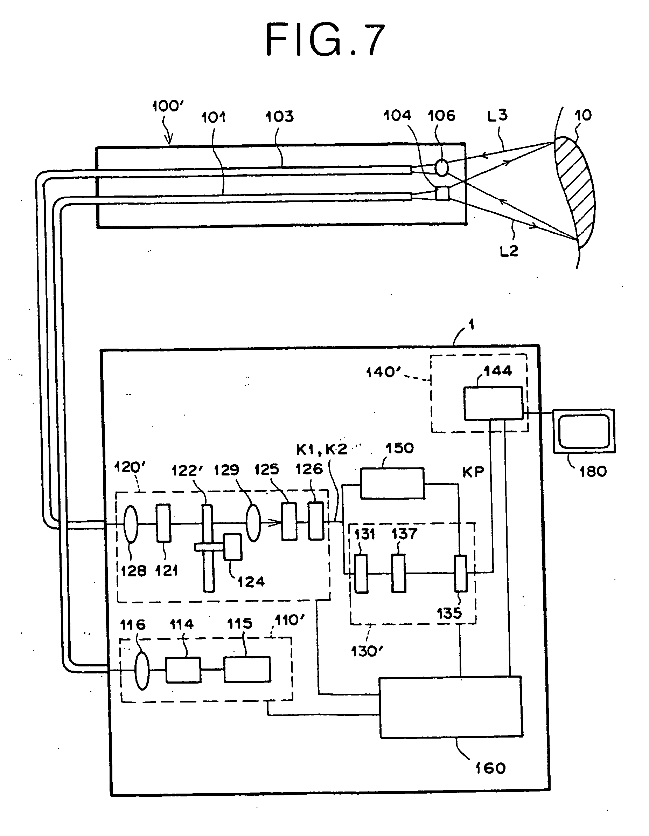

Further, according to the embodiment described above, the obtainment of a standard image, an IR reflectance image, and fluorescence images is performed, however, as shown in Fig. 7, even if the fluorescence endoscope apparatus comprises only: an endoscope insertion portion 100' provided with only a light guide 101, an image fiber 103, an illuminating lens 104, and a condensing lens 106; an illuminating unit 110' provided with only a GaN type semiconductor laser 114, an excitation light power source 115, and an excitation light condensing lens 116; an image obtaining unit 120' provided with a switching filter 122', which has only an optical filters 123a and 123b, instead of the switching filter 122; a fluorescence diagnostic image forming means 130' formed only of an image memory 131, a computed fluorescence value obtaining portion 137, and an exceptional display process portion 135 for subjecting the obstructing portions of the computed image represented by the computed fluorescence values to obtain a processed fluorescence image data KP; an image process portion 140' formed provided with only a video processing circuit 144; a controller 160; and a monitor 180 for displaying the fluorescence diagnostic image; wherein, only fluorescence images are obtained and the computed fluorescence values thereof obtained, and said computed fluorescence values displayed as fluorescence diagnostic images, the obstructing regions can be subjected to the exceptional display process and displayed in the same manner as in the second embodiment.

Further, according to the embodiments described above, the CCD imaging element for obtaining fluorescence images has been provided within the image processing portion; however, a CCD imaging element equipped with the on-chip mosaic filter can be disposed in the distal end of the endoscope insertion portion. In addition, if the CCD imaging element is a charge multiplying type CCD imaging element, such as that described in Japanese Unexamined Patent Publication No. 7 (1995) -176721, for amplifying the obtained signal charge, the obtainment of the fluorescence images can be performed at a higher sensitivity, and the noise component of the fluorescence images can be further reduced.

## Claims

1. A fluorescence image obtaining apparatus comprising: a fluorescence diagnostic image obtaining means (120) for obtaining, based on the fluorescence obtained from a target subject (10) upon the irradiation thereof by an illuminating light containing excitation light (22), a fluorescence diagnostic image of a target subject (10), further comprising an obstructing regions detecting means (151) for detecting the obstructing factors such as blood, mucus, digestive fluids, saliva, foam and the like present on the target subject, said obstructing regions detecting means (150) being a means for obtaining the fluorescence data of the target subject, based on the fluorescence, and detecting the obstructing regions based on said fluorescence data,
   **characterized in that**
   the fluorescence intensity and a computed fluorescence value representing the ratio between a plurality of fluorescence intensities (K1, K2) obtained of different wavelength bands are used as the fluorescence data.

2. A fluorescence image obtaining apparatus as defined in claim 1, wherein said obstructing regions detecting means is a means for detecting, based on either the fluorescence intensity or the computed fluorescence value, the suspected obstructing regions of the target subject, and detecting, based on the other of either of the fluorescence intensity and the computed fluorescence value of said suspected obstructing regions, the obstructing regions.

3. A fluorescence image obtaining apparatus as defined in claim 1 or 2, further comprising an exceptional display process means (135) for subjecting the obstructing regions of the fluorescence diagnostic image to exceptional display processes, and a display means for displaying the fluorescence diagnostic image that has been subjected to said exceptional display processes.

**4.** A fluorescence image obtaining apparatus as defined in claim 1 or 2, wherein a portion or the entirety of the fluorescence diagnostic image obtaining means (120) be provided in the form of an endoscope to be inserted into the body cavity of a patient.

**5.** A program for causing a computer to execute a fluorescence image obtaining method of projecting an illuminating light containing excitation light onto a target subject and obtaining a fluorescence diagnostic image based on the fluorescence obtained from said target subject upon the irradiation thereof by said light, further comprising the procedure of detecting the obstructing regions representing an obstructing factor such as blood, mucus, digestive fluids, saliva, foam and the like present on the target subject, obtaining the fluorescence data of the target subject, based on the fluorescence, and detecting the obstructing regions, based on said fluorescence data, **characterized in that** the fluorescence intensity and computed fluorescence value representing the ratio between a plurality of fluorescence intensities (K1, K2) obtained of different wavelength bands are used as the fluorescence data.

**6.** A program as defined in claims 5, further comprising the procedures of subjecting the obstructing regions of the fluorescence diagnostic image to an exceptional display process, and displaying the fluorescence diagnostic image subjected to said exceptional display process.

**Patentansprüche**

**1.** Fluoreszenzbild-Erstellungsvorrichtung, umfassend:

eine Fluoreszenz-Diagnosebild-Erstellungseinrichtung (120), die basierend auf der von einem Zielobjekt (10) bei dessen Bestrahlung mit Anregungslicht (22) enthaltendem Beleuchtungslicht erhaltenen Fluoreszenz ein Fluoreszenz-Diagnosebild des Zielobjekts (10) erstellt, weiterhin umfassend eine Sperrzonen-Detektiereinrichtung (151) zum Detektieren von Sperreinflüssen wie zum Beispiel Blut, Schleim, Verdauungsfluiden, Speichel, Schaum und dergleichen, die sich an dem Zielobjekt befinden, wobei die Sperrzonen-Detektiereinrichtung (150) eine Einrichtung ist zum Gewinnen der Fluoreszenzdaten des Zielobjekts basierend auf der Fluoreszenz und zum Detektieren der Sperrzonen basierend auf den Fluoreszenzdaten, **dadurch gekennzeichnet, dass** die Fluoreszenzintensität und ein berechneter Fluoreszenzwert, der das Verhältnis zwischen mehreren in verschiedenen Wellenlängenbändern erhaltenen Fluoreszenzintensitäten (K1, K2) repräsentiert, als die Fluoreszenzdaten verwendet werden.

**2.** Vorrichtung nach Anspruch 1, bei der die Sperrzonen-Detektiereinrichtung eine Einrichtung ist, die basierend auf entweder der Fluoreszenzintensität oder dem berechneten Fluoreszenzwert die verdächtigen Sperrzonen des Zielobjekts detektiert und basierend auf dem berechneten Fluoreszenzwert bzw. der Fluoreszenzintensität der verdächtigen Sperrzonen die Sperrzonen detektiert.

**3.** Vorrichtung nach Anspruch 1 oder 2, weiterhin umfassend eine Ausnahme-Anzeigeverarbeitungseinrichtung (135), die die Sperrzonen des Fluoreszenz-Diagnosebilds Ausnahme-Anzeigeverarbeitungen unterzieht, und eine Anzeigevorrichtung zum Anzeigen des Fluoreszenz-Diagnosebilds, welches den Ausnahme-Anzeigeverarbeitungen unterzogen wurde.

**4.** Vorrichtung nach Anspruch 1 oder 2, bei der ein Teil oder die Gesamtheit der Fluoreszenz-Diagnosebild-Erstellungseinrichtung (120) in Form eines Endoskops ausgebildet ist, welches in den Körperhohlraum eines Patienten einführbar ist.

**5.** Programm zum Veranlassen eines Computers, ein Fluoreszenzbild-Erstellungsverfahren auszuführen, bei dem ein Anregungslicht enthaltendes Beleuchtungslicht auf ein Zielobjekt projiziert wird und basierend auf der von dem Zielobjekt bei dessen Bestrahlung mit dem Licht erhaltenen Fluoreszenz ein Fluoreszenz-Diagnosebild erstellt wird, weiterhin umfassend die Prozedur des Detektierens von Sperrzonen, die einen Sperreinfluss wie zum Beispiel Blut, Schleim, Verdauungsfluide, Speichel, Schaum und dergleichen an dem Zielobjekt bilden, das Erstellen der Fluoreszenzdaten des Zielobjekts basierend auf der Fluoreszenz, und das Detektieren der Sperrzonen basierend auf den Fluoreszenzdaten, **dadurch gekennzeichnet, dass** die Fluoreszenzintensität und ein berechneter Fluoreszenzwert, der das Verhältnis zwischen mehreren, bei unterschiedlichen Wellenlängenbändern erhaltenen Fluoreszenzintensitäten (K1, K2) repräsentiert, als Fluoreszenzdaten verwendet werden.

**6.** Programm nach Anspruch 5, weiterhin umfassend die Prozeduren, mit denen die Sperrzonen des Fluoreszenz-Diagnosebilds einem Sonderanzeigeprozess unterzogen werden und das Fluoreszenz-Diagnosebild, welches dem Sonderanzeigeprozess unterzogen wurde, zur Anzeige gebracht wird.

**Revendications**

1. Appareil d'obtention d'image par fluorescence comprenant : un moyen d'obtention d'image de diagnostic par fluorescence (120) pour obtenir, sur la base de la fluorescence obtenue depuis un sujet cible (10) lors de l'irradiation de celui-ci par une lumière d'illumination contenant de la lumière d'excitation (22), une image de diagnostic par fluorescence d'un sujet cible (10), comprenant en outre un moyen de détection de régions d'obstruction (151) destiné à détecter des facteurs d'obstruction, tels que le sang, du mucus, des fluides digestifs, de la salive, de la mousse et analogue, présents sur le sujet cible, ledit moyen de détection de régions d'obstruction (150) étant un moyen destiné à obtenir les données de fluorescence du sujet cible, sur la base de la fluorescence et à détecter les régions d'obstruction sur la base desdites données de fluorescence,

   **caractérisé en ce que** l'intensité de fluorescence et une valeur de fluorescence calculée représentant un rapport entre une pluralité d'intensité de fluorescence (K1, K2) obtenues à différentes bandes de longueurs d'ondes sont utilisées comme les données de fluorescence.

2. Appareil d'obtention d'image par fluorescence selon la revendication 1, dans lequel ledit moyen de détection de régions d'obstruction est un moyen destiné à détecter, sur la base de l'intensité de fluorescence ou de la valeur de fluorescence calculée, les régions d'obstruction suspectées du sujet cible et à détecter, sur la base de l'autre de l'intensité de fluorescence et de la valeur de fluorescence calculée desdites régions d'obstruction suspectées, les régions d'obstruction.

3. Appareil d'obtention d'image par fluorescence selon la revendication 1 ou 2, comprenant en outre un moyen de traitement d'affichage exceptionnel (135) destiné à soumettre les régions d'obstruction de l'image de diagnostic par fluorescence à des traitements d'affichage exceptionnels et un moyen d'affichage destiné à afficher l'image de diagnostic par fluorescence qui a été soumise auxdits traitements d'affichage exceptionnels.

4. Appareil d'obtention d'image par fluorescence selon la revendication 1 ou 2, dans lequel une partie ou la totalité du moyen d'obtention d'image de diagnostic par fluorescence (120) est prévu sous la forme d'un endoscope à insérer dans la cavité corporelle d'un patient.

5. Programme destiné à amener un ordinateur à exécuter un procédé d'obtention d'image par fluorescence consistant à projeter une lumière d'illumination contenant de la lumière d'excitation sur un sujet cible et à obtenir une image de diagnostic par fluorescence sur la base de la fluorescence obtenue depuis ledit sujet cible lors de l'irradiation de celui-ci par ladite lumière, comprenant en outre la procédure consistant à détecter les régions d'obstruction représentant un facteur d'obstruction tel que du sang, du mucus et analogue présent sur le sujet cible, obtenir les données de fluorescence du sujet cible, sur la base de la fluorescence et détecter les régions d'obstruction, sur la base desdites données de fluorescence, **caractérisé en ce que**

   l'intensité de fluorescence et la valeur de fluorescence calculée représentant le rapport entre une pluralité d'intensités de fluorescence (K1, K2) obtenues à différentes bandes de longueurs d'ondes sont utilisées comme les données de fluorescence.

6. Programme selon la revendication 5, comprenant en outre les procédures consistant à soumettre les régions d'obstruction de l'image de diagnostic par fluorescence à un traitement d'affichage exceptionnel et afficher l'image de diagnostic par fluorescence soumise auxdits traitements d'affichage exceptionnels.

# FIG.1

# FIG.2

| G | C | G | C | G | C | G | C |
|---|---|---|---|---|---|---|---|
| G | Y | G | Y | G | Y | G | Y |
| G | C | G | C | G | C | G | C |
| Y | G | Y | G | Y | G | Y | G |
| G | C | G | C | G | C | G | C |
| G | Y | G | Y | G | Y | G | Y |
| G | C | G | C | G | C | G | C |
| Y | G | Y | G | Y | G | Y | G |

# FIG.3

# FIG.4

START

S1
OBTAIN STANDARD
IMAGE COLOR DATA

S2
OUTSIDE RANGE? — NO

YES

S4
EXCEPTIONAL
DISPLAY PROCESS

S3
NO PROCESS

END

# FIG.5

# FIG.6

```
        ┌──────────┐
        │  START   │
        └────┬─────┘
             │              S11
      ┌──────▼──────────────┐
      │  OBTAIN CONPUTED    │
      │  FLUORESCENCE VALUE │
      └──────┬──────────────┘
             │              S12
        ╱────▼─────────────╲        NO
      ◁  COMPUTED FLUORESCENCE ▷──────────┐
        ╲  VALUE ≦ Th1?    ╱              │
             │ YES                        │
             │              S14           │
        ╱────▼─────────────╲   NO         │
      ◁  FLUORESCENCE IMAGE  ▷────────┐   │
        ╲ PIXEL VALUES ≧ Th2? ╱       │   │
             │ YES                    │   │
             │          S15           ▼   ▼    S13
      ┌──────▼──────────┐          ┌────────────┐
      │  EXCEPTIONAL    │          │ NO PROCESS │
      │  DISPLAY PROCESS│          └─────┬──────┘
      └──────┬──────────┘                │
             │                           │
             ◄───────────────────────────┘
        ┌────▼─────┐
        │   END    │
        └──────────┘
```

# FIG.7

FIG.. 8

NORMAL TISSUE

DISEASED TISSUE

INTENSITY

400 450 500 550 600 650 700 750

WAVELENGTH(nm)

**9**

FIG.

**10**

FIG.

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5590660 A
- US 5647368 A
- JP 9308609 A
- JP 10225436 A

- JP 2001157658 A
- WO 9937204 A
- JP 7176721 A